# EUROPÄISCHE PATENTSCHRIFT

(11) **EP 2 247 339 B1**
(45) Veröffentlichungstag und Bekanntmachung des Hinweises auf die Patenterteilung: **02.04.2014**
(21) Anmeldenummer: 09714565.0
(22) Anmeldetag: 27.02.2009
(51) Int. Cl.: A61N 5/10

(54) **PROTONENSTRAHL-THERAPIESYSTEM**
PROTON BEAM TREATMENT SYSTEM
SYSTÈME DE THÉRAPIE PAR UN FAISCEAU DE PROTONS

(30) Priorität: 27.02.2008 DE 102008011326
(43) Veröffentlichungstag der Anmeldung: 10.11.2010
(73) Patentinhaber: CRYOELECTRA GMBH, 42287 Wuppertal (DE)
(72) Erfinder: DREES, DR., Jürgen, 42287 Wuppertal (DE); PIEL, DR., Helmut, 42287 Wuppertal (DE)
(74) Vertreter: Cohausz & Florack
(86) Internationale Anmeldenummer: PCT/EP2009/052350
(87) Internationale Veröffentlichungsnummer: WO 2009/106603

(56) Entgegenhaltungen:
- GRIESMAYER ET AL: "The MedAustron project" NUCLEAR INSTRUMENTS & METHODS IN PHYSICS RESEARCH, SECTION - B:BEAM INTERACTIONS WITH MATERIALS AND ATOMS, ELSEVIER, AMSTERDAM, NL, Bd. 258, Nr. 1, 14. April 2007 (2007-04-14), Seiten 134-138, XP022037144 ISSN: 0168-583X
- BENEDIKT ET AL: "Optics design of the extraction lines for the MedAustron hadron therapy centre" NUCLEAR INSTRUMENTS & METHODS IN PHYSICS RESEARCH, SECTION - A:ACCELERATORS, SPECTROMETERS, DETECTORS AND ASSOCIATED EQUIPMENT, ELSEVIER, AMSTERDAM, NL, Bd. 539, Nr. 1-2, 21. Februar 2005 (2005-02-21), Seiten 25-36, XP025295142 ISSN: 0168-9002 [gefunden am 2005-02-21]
- BRAHME A ET AL: "Design of a centre for biologically optimised light ion therapy in Stockholm" NUCLEAR INSTRUMENTS & METHODS IN PHYSICS RESEARCH, SECTION - B:BEAM INTERACTIONS WITH MATERIALS AND ATOMS, ELSEVIER, AMSTERDAM, NL, Bd. 184, Nr. 4, 1. Dezember 2001 (2001-12-01), Seiten 569-588, XP004313116 ISSN: 0168-583X
- AMALDI U ET AL: "The Italian project for a hadrontherapy centre" NUCLEAR INSTRUMENTS & METHODS IN PHYSICS RESEARCH, SECTION - A:ACCELERATORS, SPECTROMETERS, DETECTORS AND ASSOCIATED EQUIPMENT, ELSEVIER, AMSTERDAM, NL, Bd. A360, Nr. 1, 1. Juni 1995 (1995-06-01), Seiten 297-301, XP004009273 ISSN: 0168-9002
- FOR THE PROSCAN TEAM SCHIPPERS ET AL: "The SC cyclotron and beam lines of PSI's new protontherapy facility PROSCAN" NUCLEAR INSTRUMENTS & METHODS IN PHYSICS RESEARCH, SECTION - B:BEAM INTERACTIONS WITH MATERIALS AND ATOMS, ELSEVIER, AMSTERDAM, NL, Bd. 261, Nr. 1-2, 19. Juli 2007 (2007-07-19), Seiten 773-776, XP022152033 ISSN: 0168-583X

## Beschreibung

Die Erfindung betrifft ein Protonenstrahl-Therapiesystem mit einer Strahlerzeugungseinheit zur Erzeugung und Einstellung der kinetischen Energie eines Protonenstrahls, mit wenigstens zwei jeweils eine rotierbare isozentrische Gantry aufweisenden Strahlführungseinheiten zur Zuführung des Protonenstrahls an einen der jeweiligen Strahlführungseinheit zugeordneten Behandlungsort, wobei jede Strahlführungseinheit wenigstens eine Strahlablenkeinheit und/oder wenigstens eine Strahlformungseinheit umfasst. Die Erfindung betrifft ferner ein Verfahren zur rechnergestützten Steuerung eines solchen Protonenstrahl-Therapiesystems.

Teilchenstrahl-Therapiesysteme sind aus dem Stand der Technik, beispielsweise aus der Patentanmeldung US 2005/0139787 A1, bekannt. Typischerweise wird dabei ein Teilchenstrahl in einer Beschleunigereinrichtung erzeugt und über Strahlführungseinheiten mit magnetoptischen Einrichtungen einem von mehreren Behandlungsräumen zugeführt. Die magnetoptischen Einrichtungen in den einzelnen Behandlungsräumen sind dabei weitgehend identisch und so ausgelegt, dass sie Teilchenstrahlen mit einem möglichst breiten Spektrum an Teilchenenergie (beispielsweise von niedrigen Teilchenenergien wie etwa 70 MeV bis zu hohen Energien von etwa 250 MeV; 1 MeV entspricht ungefähr 1,6 * 10⁻¹³ Joule) mit möglichst hohem Transmissionsgrad dem Behandlungsort zuführen können. In den Behandlungsräumen kann ein Patient beispielsweise zum Zweck der Tumortherapie der Der Transmissionsgrad des Teilchenstrahls von der Beschleunigereinrichtung zu dem jeweiligen Behandlungsort ist einerseits wesentlich von der Akzeptanz der jeweiligen magnetoptischen Einrichtung als auch von der Phasenraumdichte des Teilchenstrahls abhängig. Wird der Teilchenstrahl insbesondere durch eine Beschleunigereinrichtung mit konstanter Energie erzeugt, z.B. einem Zyklotron, kann es notwendig werden, die Teilchen von der beschleunigertechnisch vorgegebenen kinetischen Energie beispielsweise mittels eines Degraders auf eine geringere kinetische Energie abzubremsen. Durch solche Abbremsprozesse wird jedoch die Phasenraumdichte der Teilchen im Teilchenstrahl verringert, was die Anforderungen an die Akzeptanz der nachfolgenden magnetoptischen Einrichtungen zur Strahlführung und/oder Strahlformung erhöht.

Die Bereitstellung mehrerer weitgehend identische Strahlführungseinheiten aufweisender Behandlungsräume nebeneinander hat insbesondere den Vorteil, dass Behandlungspläne, welche eine Mehrzahl von zu behandelnden Patienten umfassen, flexibel und zeiteffizient aufgestellt werden können. So ist es beispielsweise möglich, nach der vollzogenen Behandlung eines ersten Patienten in einem ersten Behandlungsraum, diesen ersten Behandlungsraum für die nächste Behandlung vorzubereiten, während gleichzeitig ein weiterer Patient in einem der anderen vorbereiteten Behandlungsräume dem Teilchenstrahl aus der gleichen Beschleunigereinrichtung ausgesetzt wird. Die Bereitstellung mehrerer weitgehend identische Strahlführungseinheiten aufweisender Behandlungsräume weist demnach insofern einen Kostenvorteil auf, da die Anzahl der zu behandelnden Patienten pro Zeit erhöht werden kann.

Um den Teilchenstrahl mit einem für eine Behandlung hinreichenden Transmissionsgrad von der Strahlerzeugungseinheit zu dem Behandlungsort zu führen, werden hohe vorrichtungstechnische und betriebliche Anforderungen an die Strahlführungseinheiten gestellt. Dies gilt insbesondere für den Teil der Strahlführungseinheit, welcher in einem Behandlungsraum selbst angeordnet ist. Die benötigten magnetoptischen Einrichtungen zur Strahlführung und/oder Strahlformung sind dort zumeist an einer Gantry mit beweglicher Stelleinrichtung montiert, um gewährleisten zu können, dass ein im Behandlungsraum gelagerter Patient aus möglichst vielen unterschiedlichen Richtungen dem Teilchenstrahl ausgesetzt werden kann. Zu diesem Zweck sind die Gantrys mittels der Stelleinrichtung üblicherweise um eine horizontale Achse um bis zu 360° drehbar in dem Behandlungsraum montiert.

Eine über weite Strahlenergiebereiche reichende Strahlführung erfordert eine magnetoptische Einrichtung mit diversen multipolaren (Elektro-)Magneten, beispielsweise Dipolmagneten, Quadrupolmagneten und gegebenenfalls Sextupolmagneten, welche eine dem Zweck angepasste räumliche Ausdehnung und eine entsprechende Masse besitzen. Darüber hinaus müssen die (Elektro-)Magnete mit über weite Bereiche regelbaren Energieversorgungen verbunden sein, um die zur Strahlführung erforderlichen magnetischen Feldstärken erzeugen zu können. Insbesondere das Wegintegral über die magnetische Feldstärke bestimmt das Strahlführungsvermögen der magnetoptischen Einrichtung wesentlich mit. Zur Führung von Strahlen mit hohen Teilchenenergien sind auch entsprechend hohe magnetische Feldstärken notwendig, welche nur mit entsprechend ausgebildeten (Elektro-)Magneten erzeugt werden können, wohingegen die Anforderungen an das Wegintegral über die magnetische Feldstärke bei Strahlen mit niedrigen Teilchenenergien proportional zum Impuls der Teilchen verringert sind.

Auf Grund des hohen vorrichtungstechnischen Aufwands für solche magnetoptische Einrichtungen müssen auch hohe Anforderungen an die Gantry und deren Stelleinrichtung gestellt werden, weil diese genügend Raum für die Magnete zur Verfügung stellen und ausreichend Stellkraftpotenzial aufweisen müssen, um die schweren magnetoptischen Einrichtungen schnell und präzise rotieren zu können. Um ein Beispiel für mögliche Abmessungen zu nennen, kann eine Gantry eine Länge von etwa zehn Metern und eine Breite von etwa vier Metern aufweisen. Folglich benötigt der Behandlungsraum, in welchem die Gantry montiert ist, auch eine hohe Tragfähigkeit, um die Gantry weitgehend erschütterungsfrei zu halten. Der durch die hohe Flexibilität bei der Erstellung der Behandlungspläne erlangte Kostenvorteil wird durch den vorrichtungstechnischen und betrieblichen Aufwand für die einzelnen instrumentell weitgehend identisch ausgebildeten magnetoptischen Einrichtungen und Gantrys wieder vermindert.

Die Technik, welche der Protonenstrahl-Therapie zu Grunde liegt, ist auch in den folgend genannten wissenschaftlichen Veröffentlichungen näher beschrieben:
- Karl L. Brown, Sam K. Howry, "TRANSPORT, A Computer Program for Designing Charged Particle Beam Transport Systems", SLAC Report No. 91 (1970) and later updates of the TRANSPORT program by U. Rohrer and others
- U. Rohrer, "PSI Graphic TURTLE Framework based on a CERN-SLAC-FERMILAB version by K.L. Brown et al.", http://people.web.psi.ch/rohrer-u/turtle.htm (2008)
- J. Drees, "Passage of Protons through Thick Degraders", Cryoelectra Report Sept. 2008

In der Veröffentlichung "The SC cyclotron and beam lines of PSI's new protontherapy facility PROSCAN" von J.M. Schippers et al., Nuclear Instruments and Methods in Physics Research, Elsevier, Amsterdam, Band 261, Nr. 1-2, 19. Juli 2007, S. 773-776, wird ein Protonenstrahl-Therapiesystem mit zwei Gantries gemäß dem Oberbegriff des Patentanspruchs 1 beschrieben.

Hiervon ausgehend liegt der vorliegenden Erfindung die Aufgabe zu Grunde, ein Teilchenstrahl-Therapiesystem anzugeben, mit welchem der vorrichtungstechnische, betriebliche Aufwand und damit verbunden auch der Kostenaufwand reduziert werden können. Weiterhin liegt der vorliegenden Erfindung die Aufgabe zu Grunde, ein Verfahren zur Steuerung eines solchen Teilchenstrahl-Therapiesystems vorzuschlagen.

Gemäß einer ersten Lehre der vorliegenden Erfindung wird die Aufgabe mit einem Protonenstrahl-Therapiesystem gemäß dem Oberbegriff des Patentanspruchs 1 dadurch gelöst, dass die Strahlführungseinheiten instrumentell unterschiedlich ausgelegt sind, derart, dass sie für unterschiedliche Bereiche der kinetischen Energie der Protonen und/oder für unterschiedliche Strahleigenschaften ausgelegt sind. Die Strahlführungseinheiten sind dazu derart ausgeführt, dass die wenigstens eine Strahlablenkeinheit und/oder wenigstens eine Strahlformungseinheit einer jeden Strahlführungseinheit an den jeweiligen Bereich der die Energie der Protonen und/oder die jeweilige Strahleigenschaft des Protonenstrahls angepasst sind.

Dem hier beschriebenen Protonenstrahl-Therapiesystem liegt die Überlegung zu Grunde, die Variabilität der für verschiedene Protonenstrahl-Therapievorgänge erforderlichen Strahlenergie für eine instrumentelle bzw. vorrichtungstechnische Vereinfachung des Protonenstrahl-Therapiesystems auszunutzen (mit anderen Worten, nicht für jeden Behandlungsvorgang ist die Einstellung der Energie der Teilchen über den maximal möglichen Energiebereich notwendig). Es ist demnach nicht mehr erforderlich, die einzelnen Strahlführungseinheiten mit ihren Strahlablenkeinheiten und/oder Strahlformungseinheiten weitgehend identisch auszubilden, so dass ein Protonenstrahl beliebiger Energie von jeder der vorhandenen Strahlführungseinheiten, weitgehend optimal (also mit größtmöglichem Transmissionsgrad), führbar ist. Vielmehr werden den Strahlführungseinheiten unterschiedliche Protonenstrahl-Energien bzw. Strahleigenschaften zugeordnet, und die Strahlführungseinheiten werden dementsprechend instrumentell unterschiedlich ausgelegt. Daraus resultiert eine Vereinfachung der einzelnen Strahlführungseinheiten.

Ein aus der Strahlerzeugungseinheit hervorgehender Protonenstrahl wird gemäß der Energie seiner Protonen und/oder gemäß seiner Strahleigenschaften nicht irgendeiner Strahlführungseinheit aus einer Mehrheit von instrumentell weitgehend identisch ausgestatteten Strahlführungseinheiten zugeführt, sondern einer Strahlführungseinheit, deren magnetoptische Einrichtung, insbesondere deren Strahlablenkeinheit und/oder Strahlformungseinheit, speziell auf die Führung eines Strahls von Protonen dieser Energie und/oder mit diesen Strahleigenschaften ausgelegt, insbesondere optimiert, ist.

Auf diese Weise können die Anforderungen an die einzelnen Strahlführungseinheiten verringert werden. Die Multipolmagnete einer solchen "spezialisierten" Strahlführungseinheit, insbesondere deren Strahlablenkeinheit und/oder Strahlformungseinheit, können beispielsweise weniger voluminös und mit geringerem Gewicht ausgeführt werden. Zusätzlich oder alternativ kann gegebenenfalls auch die Energieversorgung der (Elektro-)Magnete der magnetoptischen Einrichtung kleiner dimensioniert und damit vereinfacht werden. Die Gantry-Stelleinrichtung der derart vereinfachten magnetoptischen Einrichtung kann somit ebenfalls geringer dimensioniert und gegebenenfalls leichter manövriert, also um den Behandlungsort gedreht, werden. Eine Kostenreduktion für das Protonenstrahl-Therapiesystem als Ganzes ergibt sich damit aus der Reduzierung des instrumentellen Aufwands für jede einzelne Strahlführungseinheit (inklusive Strahlablenkeinheit, Strahlformungseinheit und gegebenenfalls Energieversorgung und/oder Gantry).

In einer bevorzugten Ausgestaltung des Protonenstrahl-Therapiesystems können die wenigstens zwei Strahlführungseinheiten unterschiedliche Akzeptanzen aufweisen. Die Akzeptanz ist eine aus der Beschleunigerphysik bekannte Größe und definiert den maximal möglichen mehrdimensionalen Phasenraum, welchen eine Strahlführungseinheit zum zielort, hier beispielsweise dem Behandlungsort, transportieren kann. Der Phasenraum repräsentiert die räumliche Ausdehnung des Protonenstrahls, dessen Divergenzverhalten und dessen Impulsvarianz. Bei Abbremsung des primären Protonenstrahls, z.B. durch einen Degrader, weitet sich der von den Protonen eingenommene Phasenraum stark auf. Die Phasenraumdichte sinkt umso stärker, je stärker die Energie reduziert wird.

Folglich kann wenigstens eine der Strahlführungseinheiten instrumentell auf eine hohe Akzeptanz ausgelegt sein. Diese Strahlführungseinheit wäre dann insbesondere für Strahlen mit niedrigen Protonenenergien geeignet. Hingegen kann wenigstens eine aus der Mehrzahl der Strahlführungsvorrichtungen auf geringere Akzeptanzen ausgelegt werden, um den vorrichtungstechnischen Aufwand des Protonenstahl-Therapiesystems als Ganzes zu reduzieren. Die auf niedrige Akzeptanzen ausgelegte Strahlführungseinheit wäre demnach insbesondere für Strahlen mit hohen Protonenenergien geeignet.

Weiterhin kann wenigstens eine der Strahlführungseinheiten auf eine optimale Strahltransmission ausgelegt sein. In dieser vorteilhaften Ausgestaltung des Protonenstrahl-Therapiesystems wird die Strahlführungseinheit auf einen möglichst geringen Verlust an Protonen bzw. Strahlintensität durch die Strahlführung ausgelegt (mit anderen Worten, auf einen größtmöglichen Transmissionsgrad), Dies kann insbesondere dann vorteilhaft sein, wenn der Protonenstrahl beispielsweise auf Grund einer großen räumlichen Ausdehnung, insbesondere mit einem breiten Strahlprofil, eine niedrige Phasenraumdichte aufweist.

Zusätzlich oder alternativ kann wenigstens eine der Strahlführungseinheiten auf eine optimale Strahlfokussierung ausgelegt sein. In dieser vorteilhaften Ausgestaltung des Protonenstrahl-Therapiesystems wird die Strahlführungseinheit darauf ausgelegt, einen stark fokussierten Protonenstrahl mit entsprechend hoher Energiedichte pro Fläche am Behandlungsort abzubilden. Mit einer solchen Ausgestaltung einer der Strahlführungseinheiten kann das räumliche Auflösungsvermögen in wenigstens einem Behandlungsraum gesteigert werden, beispielsweise um die Beeinträchtigung der um den Behandlungsort gelegenen Volumina durch unbeabsichtigte Bestrahlung zu vermindern.

Durch die Auslegung wenigstens einer Strahlführungseinheit auf optimale Strahltransmission und zusätzlich oder alternativ auf Strahlfokussierung kann die Einschaltzeit der Beschleunigereinrichtung verkürzt werden. Auf diese Weise kann auch die kumulierte radioaktive Belastung der Räumlichkeiten, in welchen sich das Protonenstrahl-Therapiesystem befindet, reduziert werden.

Der Phasenraum des Protonenstrahls wird insbesondere durch Ort und Impuls der Teilchen bestimmt. Je höher die Phasenraumdichte ist, desto schmaler ist im Regelfall das Strahlprofils und desto geringer die Divergenz des Protonenstrahls. Ein Protonenstrahl mit hoher Phasenraumdichte erfordert einen geringen Strahlführungsaufwand im vergleich zu einem Protonenstrahl mit geringer Phasenraumdichte, weil die Varianz der Phasenraumparameter im letztgenannten Fall höher ist.

Demzufolge können die Strahlführungseinheiten sich instrumentell dadurch unterscheiden, dass wenigstens eine von ihnen für Protonenstrahlen mit hoher Phasenraumdichte ausgelegt ist, wodurch sich der vorrichtungstechnische Aufwand für diese Strahlführungseinheit verringert, oder dass wenigstens eine von ihnen für Protonenstrahlen mit geringer Phasenraumdichte ausgelegt ist. Im letztgenannten Fall kann der instrumentelle Aufwand nicht beliebig variiert werden, sorgt aber dafür, dass auch ein Protonenstrahl mit geringer Phasenraumdichte mit einem ausreichenden Transmissionsgrad dem Behandlungsort effizient zugeführt werden kann.

In einer speziellen Ausgestaltung des Protonenstrahl-Therapiesystems kann die Strahlerzeugungseinheit eine Beschleunigereinrichtung mit einstellbarer kinetischer Energie, insbesondere ein Synchrotron, aufweisen. Bei einem Synchrotron kann die Energie des Protonenstrahls in einem weiten Bereich eingestellt werden. Da sich diese sehr präzise einstellen lässt, weist ein mit einem Synchrotron erzeugter Protonenstrahl üblicherweise eine hohe Phasenraumdichte auf.

In einer alternativen Ausgestaltung des Protonenstrahl-Therapiesystems kann die Strahlerzeugungseinheit eine Beschleunigereinrichtung mit konstanter kinetischer Energie, insbesondere ein Zyklotron, und eine Energiekorrektureinheit, insbesondere einen Degrader, aufweisen. Mit einem, insbesondere supraleitenden, Zyklotron können lediglich Protonenstrahlen einer bestimmten festen Energie erzeugt werden. Dieser Energiewert wird üblicherweise recht hoch, beispielsweise bei etwa 250 MeV, angesetzt. Da nicht bei allen Behandlungsvorgängen eine derart hohe Energie der Protonen erforderlich ist - beispielsweise erfordert eine Behandlung oberflächennaher Tumore Protonen mit geringerer kinetischer Energie -, kann der Protonenstrahl nach der Erzeugung im Zyklotron eine Energiekorrektureinheit, beispielweise einen Degrader, durchlaufen, mittels derer die Energie des Protonenstrahls auf das gewünschte Maß eingestellt wird.

Der Degrader kann vorzugsweise ein Material mit niedriger Ordnungszahl Z, insbesondere Z kleiner als 10, umfassen. Durch die physikalische Wechselwirkung des Protonenstrahls mit dem Degradermaterial kann die kinetische Energie der Protonen somit abwärts auf das gewünschte Maß korrigiert werden. Ein Vorteil dieser Technik gegenüber einem Synchrotron liegt vor allem in den geringeren Betriebskosten eines Zyklotrons. Auf Grund der nicht detailliert vorhersehbaren Wechselwirkung der Protonen mit dem Degradermaterial weist ein Protonenstrahl, welcher einen Degrader durchlaufen hat, in der Regel eine geringere Phasenraumdichte auf als der Protonenstrahl unmittelbar nach dem Austritt aus einem Zyklotron (oder ein Protonenstrahl, welcher aus einem Synchrotron austritt).

Es wurde festgestellt, dass insbesondere in der Tumortherapie am Menschen die Bestrahlung mit Protonen als sehr einfachen Ionen, eine bessere Wirksamkeit erzielt als beispielsweise eine Bestrahlung mit Photonen. Weiterhin ist eine Bestrahlung mit Protonen vorteilhaft, da diese erst am Ende ihres Laufwegs im zu bestrahlenden Gewebe, im so genannten Bragg-Peak, ihre maximale Ionisationsstärke, und damit einhergehend ihre größte Zerstörungskraft, beispielsweise für Tumorzellen, aufweisen. Auf diese Weise kann die Beeinträchtigung von gesundem Gewebe, welches dem zu behandelnden Gewebe im Laufweg des Strahls vorgelagert ist und von dem Strahl durchlaufen wird, reduziert werden.

Die Strahlführungseinheiten, weisen jeweils eine bewegliche Gantry auf. Die Gantries können in vorteilhafter Weise, insbesondere um eine horizontale Achse, um bis zu 360° rotierbar sein, um den Behandlungsort aus möglichst vielen Winkeln bestrahlen zu können. Die Gantry umfasst ferner insbesondere eine daran montierte magnetoptische Einrichtung, insbesondere wenigstens eine Strahlablenkeinheit und/oder Strahlformungseinheit mit Dipolmagneten, Quadrupolmagneten und gegebenenfalls weiteren Ablenkmagneten (z.B. Sextupolmagneten,...) zur Strahlführung und Strahlformung, um den Protonenstrahl von der von der Strahlerzeugungseinheit vorgegebenen Strahlrichtung auf den Behandlungsort hin zu leiten und umzulenken.

Die magnetoptischen Einrichtungen der einzelnen Strahlführungseinheiten sind vorrichtungstechnisch bzw. instrumentell untereinander nicht identisch ausgebildet, sondern an die Energie der Protonen und/oder die Strahleigenschaften des Protonenstrahls angepasst. Dies geht einher mit einer Reduktion des vorrichtungstechnischen und betrieblichen Aufwands jeder einzelnen Strahlführungseinheit, beispielsweise der Masse und/oder der Abmessungen der Ablenkmagneten oder des Komplexitätsgrades der Energieversorgungen der (Elektro-)Magnete, wodurch der instrumentelle Aufwand des Protonenstrahl-Therapiesystems als Ganzes verringert werden kann. Dies geht einher mit einer vorteilhaften Kostenersparnis.

Gemäß einer weiteren Lehre der vorliegenden Erfindung wird die Aufgabe auch durch ein Verfahren nach Anspruch 7 zur rechnet gestützten Steuerung eines wie zuvor beschrieben ausgebildeten Protonenstrahl-Therapie systems gelöst.

Hinsichtlich der Vorteile oder vorteilhafter Ausführungsformen des Verfahrens wird auf die Ausführungen zu dem Protonenstrahl-Therapiesystem verwiesen.

Bevorzugt ist die Verwendung eines wie zuvor beschriebenen Protonenstrahl-Therapiesystems zur Bestrahlung von, insbesondere menschlichem, Gewebe.

Besonders bevorzugt ist die Verwendung eines wie zuvor beschriebenen Protonenstrahl-Therapiesystems in der Tumortherapie, insbesondere am Menschen.

In einer medizinischen Anwendung ist es besonders vorteilhaft, die Auslegung der einzelnen Strahlführungseinheiten auf unterschiedlichen Energien der Protonen und/oder Strahleigenschaften an den medizinischen Erfordernissen und der Häufigkeit der auftretenden Erkrankungsformen, welche eine Behandlung mit bestimmten Protonenenergien und/oder Strahleigenschaften indizieren, auszulegen.

Es gibt vielfältige Möglichkeiten, das Protonenstrahl-Therapiesystem oder das Verfahren gemäß der vorliegenden Erfindung auszugestalten und weiterzubilden. Hierzu wird einerseits auf die abhängigen Patentansprüche und andererseits auf die Beschreibung eines Ausführungsbeispiels in Verbindung mit der beigefügten Zeichnung verwiesen. In der Zeichnung zeigen:
- Fig. 1: ein Ausführungsbeispiel des Protonenstrahl-Therapiesystems gemäß der vorliegenden Erfindung in einer schematischen Ansicht;
- Fig. 2: eine schematische Übersicht eines Ausführungsbeispiels des Protonenstrahl-Therapiesystems gemäß der vorliegenden Erfindung.

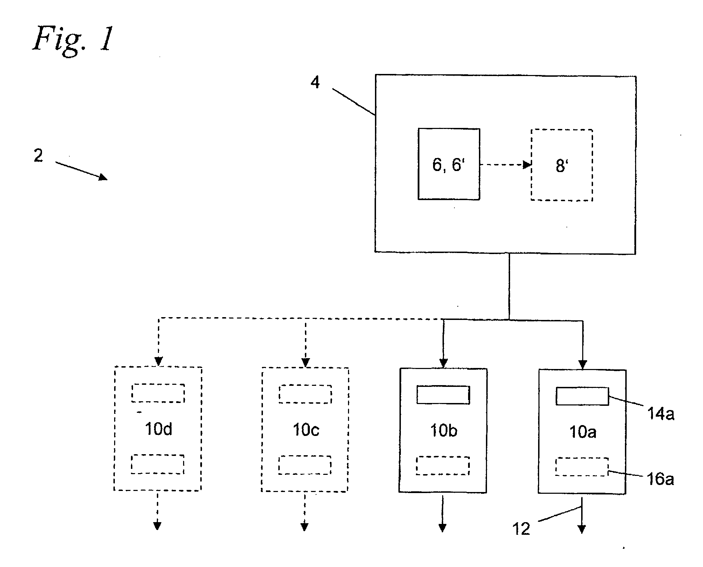

Figur 1 zeigt in einer schematischen Ansicht ein als Protonenstrahl- Therapie system ausgebildetes Teilchenstrahl-Therapiesystem 2 gemäß der vorliegenden Erfindung. Das Teilchenstrahl-Therapiesystem 2.weist eine Strahlerzeugungseinheit 4 auf. Die Strahlerzeugungseinheit 4 kann beispielsweise eine Beschleunigereinrichtung mit einstellbarer kinetischer Energie wie ein Synchrotron 6 aufweisen.

In einer alternativen Ausgestaltung des Teilchenstrahl-Therapiesystems kann die Strahlerzeugungseinheit 4 auch eine Beschleunigereinrichtung mit konstanter kinetischer Energie wie ein Zyklotron 6' aufweisen. Das Zyklotron 6' ist dann vorzugsweise auf die Bereitstellung eines Teilchenstrahls mit einer hohen Energie, beispielsweise in der Größenordnung von 200 bis 300 MeV, insbesondere 250 MeV, ausgelegt. Weil nicht für jeden Behandlungsvorgang Teilchenstrahlen einer derart hohen Energie erforderlich sind, kann dem Zyklotron 6' eine Energiekorrektureinheit nachgeordnet sein. Die Energiekorrektureinheit kann beispielsweise als Degrader 8' ausgebildet sein. Der Teilchenstrahl durchläuft dann nach dem Austritt aus dem Zyklotron 6' den Degrader 8' und wird dabei auf Grund der physikalischen Wechselwirkung der Teilchen mit dem Degradermaterial abgebremst. Durch Wahl eines Materials mit einer entsprechenden Ordnungszahl kann das Maß der Abbremsung des Teilchenstrahls in dem Degrader 8' eingestellt werden.

Nachdem der Teilchenstrahl durch die Strahlerzeugungseinheit 4 mit der gewünschten Energie versehen worden ist, wird er einer der Strahlführungseinheiten 10a, 10b zugeführt. Die Strahlführungseinheiten 10a, 10b sind dazu ausgebildet, den Teilchenstrahl einem ihnen jeweils zugeordneten Behandlungsort (Isozentrum) zuzuführen (Pfeil 12). Die Behandlungsorte befinden sich dabei vorzugweise in verschiedenen Behandlungsräumen, um eine hohe Flexibilität beispielsweise bei der Behandlung einer Mehrzahl von (Tumor- )Patienten gewährleisten zu können.

Die Strahlführungseinheiten 10a, 10b weisen in diesem Beispiel je eine Strahlablenkeinheit 14a auf, mittels derer der Teilchenstrahl in eine bestimmte Richtung auf den jeweiligen Behandlungsort hin gelenkt werden kann und von denen der Übersichtlichkeit halber nur eine mit Bezugszeichen versehen ist. Zusätzlich oder alternativ können die Strahlführungseinheiten 10a, 10b auch eine Strahlformungseinheit 16a aufweisen, mittels derer der Teilchenstrahl beispielsweise kollimiert und/oder an anderer Stelle im Strahlweg fokussiert werden kann und von denen der Übersichtlichkeit halber ebenfalls nur eine mit Bezugszeichen versehen ist. Die in Figur 1 gezeigte serielle Anordnung der Strahlablenkeinheit 14a und der Strahlformungseinheit 16a ist lediglich schematisch zu verstehen. Ebenso ist es möglich, die Reihenfolge zu vertauschen. Gleichfalls können die Strahlablenkeinheit 14a und die Strahlformungseinheit 16a mehrere Untereinheiten (nicht dargestellt) aufweisen, welche beispielsweise untereinander abwechselnd angeordnet sein können.

Die Strahlführungseinheiten 10a, 10b mit ihren Strahlablenkeinheiten 14a und Strahlformungseinheiten 16a sind untereinander nicht identisch aufgebaut. Vielmehr sind die Strahlführungseinheiten 10a, 10b für unterschiedliche Bereiche der kinetischen Energie der geladenen Teilchen des Teilchenstrahls ausgelegt. Diese unterschiedliche Auslegung kann beispielsweise unter anderem in Form unterschiedlicher Akzeptanzen bestehen. Durch die unterschiedliche Auslegung der Strahlführungseinheiten 10a, 10b wird es möglich, den von der Strahlerzeugungseinheit 4 erzeugten Teilchenstrahl einer speziell ausgebildeten Strahlführungseinheit der Strahlführungseinheiten 10a, 10b gemäß der Energie der Teilchen und/oder gemäß der Strahleigenschaften zuzuordnen. Eine rechnergestützte Steuerung (nicht gezeigt) des Teilchenstrahl-Therapiesystems 2 weist vorzugsweise einen entsprechenden zuordnungsalgorithmus auf. Die Energie der Teilchen bzw. die Strahleigenschaften können dabei entweder aus den Einstellungen der Strahlerzeugungseinheit 4 abgeleitet oder zusätzlich oder alternativ mittels entsprechender Monitore (nicht gezeigt) am Teilchenstrahl gemessen werden.

Diese unterschiedliche Zuordnung kann demnach an Hand der Energie der Teilchen erfolgen. Kann die Strahlerzeugungseinheit 4 beispielsweise Teilchen mit einer Energie zwischen 70 MeV und 250 MeV erzeugen, ist es möglich dieses Energieintervall zu unterteilen, beispielsweise in ein erstes Energieintervall von 100 bis 250 MeV und ein zweites Energieintervall von 70 bis 200 MeV, und je eine der Strahlführungseinheiten 10a, 10b vorrichtungstechnisch auf die magnetoptische Führung von Teilchen des entsprechenden Energieintervalls auszulegen. In diesem Beispiel würde ein Teilchenstrahl, welcher eine Energie von 250 MeV aufweist, der ersten Strahlführungseinheit 10a, und damit einem ersten Behandlungsort in einem ersten Behandlungsraum, zugeführt, wohingegen ein Teilchenstrahl, welcher eine Energie von weniger als 100 MeV aufweist, der zweiten Strahlführungseinheit 10b, und damit einem zweiten Behandlungsort in einem zweiten Behandlungsraum, zugeführt würde.

Für ein bestimmtes Energieintervall existiert demnach eine "spezialisierte" Strahlführungseinheit 10a, 10b. Dies ist besonders vorteilhaft, wenn für einen Behandlungsvorgang nicht die gesamte Spannbreite der möglichen Teilchenenergien, sondern nur ein beschränkter Energiebereich unterhalb der maximalen Teilchenenergie erforderlich ist. Dies erlaubt eine Reduktion des vorrichtungstechnischen Aufwands für die einzelne Strahlführungseinheit 10a, 10b und die Strahlablenkeinheiten 14a und/oder Strahlformungseinheiten 16a, beispielsweise in Form verringerter Dimensionen und verringertem Gewicht der (Elektro-)Magnete oder geringerem Komplexitätsgrad der Energieversorgungen (nicht dargestellt) der (Elektro-)Magnete. Insbesondere können die Regelbereiche der Energieversorgungen, beispielsweise Spannungs- oder Stromstärkenintervalle, verkleinert werden. Es kann somit ein kompakterer Aufbau des Teilchenstrahl-Therapiesystems 2 erzielt werden.

Es ist ebenso möglich, dass eines der Energieintervalle vollständig in einem anderen Energieintervall enthalten ist. Dies kann dann von Vorteil sein, wenn in einem eingeschränkten Energiebereich mit einem besonders hohen Patientenaufkommen und damit vielen Behandlungsfällen zu rechnen ist. Beispielsweise könnte eine Strahlführungseinheit 10a für den Energiebereich von 70 bis 250 MeV ausgelegt sein. In diesem Fall könnte die Strahlführungseinheit 10a für alle Patienten und eine andere Strahlführungseinheit 10b für eine Anzahl von Patienten eingesetzt werden. Der Vorteil des verringerten instrumentellen Aufwands würde dann durch die Vereinfachung der Strahlführungseinheit 10a, 10b mit der Auslegung auf das kleinere Energieintervall realisiert.

Die Zuordnung der Teilchenstrahlen zu den einzelnen Strahlführungseinheiten 10a, 10b kann in vorteilhafter Weise neben der Energie der Teilchen zusätzlich oder alternativ an Hand der Phasenraumdichte, der Breite des Strahlprofils oder anderen einschlägigen Strahleigenschaften erfolgen.

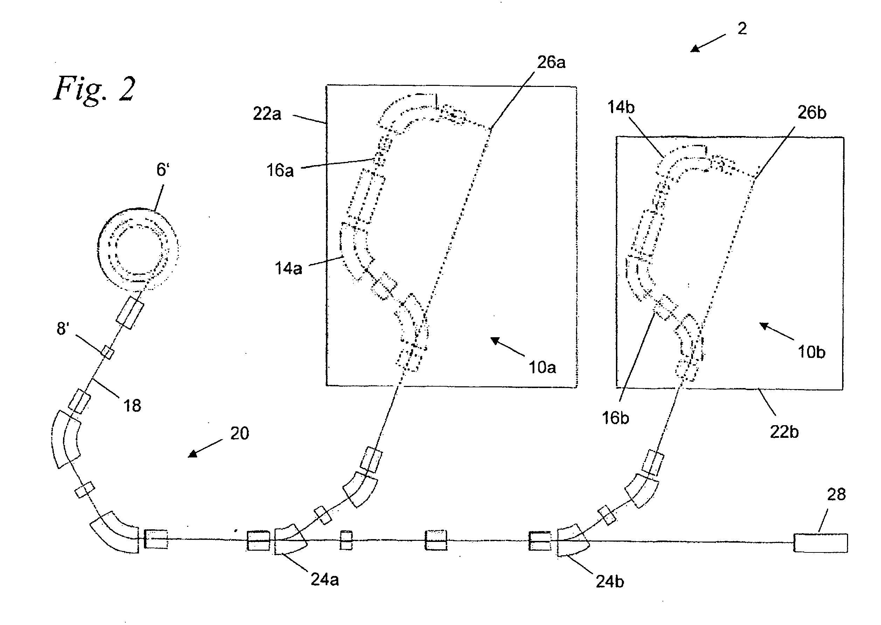

Figur 2 zeigt in einer schematischen Übersichtsdarstellung ein als Protonenstrahl-Therapie system ausgebildetes Teilchenstrahl-Therapiesystem 2. Das Teilchenstrahl-Therapiesystem 2 weist eine Beschleunigereinrichtung in Form eines Zyklotrons 6' auf. Zur Korrektur der Energie des Teilchenstrahls, sofern nicht die Maximalenergie erforderlich ist, befindet sich im Strahlweg 18 eine Energiekorrektureinheit in Form eines Degraders 8'. Nach Durchlaufen des Degraders verläuft der Strahlweg 18 des Teilchenstrahls durch eine Strahltransfereinheit 20 mit mehreren magnetoptischen Elementen, welche den Teilchenstrahl an einer seriellen Anordnung von mehreren, in diesem Beispiel zwei, Behandlungsräumen 22a, 22b vorbeiführen. An zwei Orten des Strahlwegs 18 befinden sich demnach Abzweigungseinheiten 24a, 24b, mittels derer der Teilchenstrahl in Richtung auf die einzelnen Behandlungsräume 22a, 22b umgelenkt werden kann. Hinter den Abzweigungseinheiten 24a, 24b befindet sich in der Strahlverlängerung aus Sicherheitsgründen eine Strahlauffangeinheit 28. Die Entscheidung, wann ein Teilchenstrahl welchem Behandlungsraum 22a, 22b zugeführt wird, erfolgt - wie bereits erläutert - an Hand der Energie der Teilchen und/oder der Strahleigenschaften. Im Falle von überlappenden Parameterintervallen kann aber auch der Behandlungsplan für die einzelnen Behandlungsräume 22a, 22b für die Wahl der Strahlführungseinheit 10a, 10b herangezogen werden.

In den einzelnen Behandlungsräumen 22a, 22b sind jeweils magnetoptische Einrichtungen in Form von Strahlablenkeinheiten 14a, 14b (beispielsweise zweimal um Winkel von +/- 70°, einmal um einen Winkel von 90°) und Strahlformungseinheiten 16a, 16b (zum Beispiel zur Kollimation oder Fokussierung) aufweisende Strahlführungseinheiten 10a, 10b angeordnet, welche dafür sorgen, dass nach vorgegebenen Bedingungen, beispielsweise hohem Fokussierungsgrad, der Teilchenstrahl dem jeweiligen Behandlungsort 26a, 26b zugeführt wird. Die Strahlablenkeinheiten 14a, 14b und/oder die Strahlformungseinheiten 16a, 16b sind - wie bereits ausgeführt - in vorteilhafter Weise an die Energie der Teilchen und/oder der Strahleigenschaften angepasst, so dass ein Teilchenstrahl mit bestimmter Konfiguration mit einer der Strahlführungseinheiten 10a, 10b besonders effizient geführt werden kann. Die Strahlführungseinheiten 10a, 10b weisen vorzugsweise rotierbare Gantrys auf, mittels derer die magnetoptischen Einrichtungen um den Behandlungsort 26a, 26b rotiert werden können (bis zu 360°), um unterschiedliche Bestrahlungswinkel einzustellen.

Das Teilchenstrahl-Therapiesystem ist nicht auf Ausgestaltungen mit zwei Strahlführungseinheiten 10a, 10b beschränkt. Es kann weiterhin mehr als zwei, beispielsweise wie in Figur 1 gestrichelt angedeutet vier, Strahlführungseinheiten 10a, 10b, 10c, 10d aufweisen. Auf diese Weise kann die Zuordnung der Teilchenstrahlen gemäß der Energie der Teilchen und/oder der Strahleigenschaften noch weiter differenziert werden. Eine der Anzahl der Strahlführungseinheiten 10a, 10b, 10c, 10d entsprechende Anzahl an Behandlungsräumen 22a, 22b mit darin angeordneten Behandlungsorten 26a, 26b wird dann ebenfalls bereit gestellt. Um das zuvor genannte Beispiel fortzuführen, kann eine der Strahlführungseinheiten 10a für das Energieintervall von 100 bis 250 MeV, die beiden folgenden Strahlführungseinheiten 10b, 10c für 70 bis 200 MeV und die in diesem fortgeführten Beispiel letzte Strahlführungseinheit 10d für 70 bis 150 MeV ausgelegt sein.

Es versteht sich, dass auch in diesem fortgeführten Beispiel die Zuordnung der Teilchenstrahlen zusätzlich oder alternativ an Hand der Phasenraumdichte oder der Breite des Strahlprofils erfolgen kann. Die zuvor an Hand der Energie der Teilchen erläuterten speziellen Ausgestaltungen der Zuordnung von Teilchenstrahlen zu den einzelnen Strahlführungseinheiten 10a, 10b, 10c, 10d können überdies auch bei den anderen Strahleigenschaften verwendet werden.

## Patentansprüche

1. Protonenstrahl-Therapiesystem (2)
- mit einer Strahlerzeugungseinheit (4) zur Erzeugung und Einstellung der kinetischen Energie eines Protonenstrahls,
- mit wenigstens zwei jeweils eine rotierbare isozentrische Gantry aufweisenden Strahlführungseinheiten (10a, 10b, 10c, 10d) zur Zuführung des Protonenstrahls an einen der jeweiligen Strahlführungseinheit (10a, 10b, 10c, 10d) zugeordneten Behandlungsort (26a, 26b),
- wobei die wenigstens zwei Strahlführungseinheiten (10a, 10b, 10c, 10d) jeweils wenigstens eine Strahlablenkeinheit (14a, 14b) und/oder wenigstens eine Strahlformungseinheit (16a, 16b) umfassen,
**dadurch gekennzeichnet, dass** die wenigstens zwei Strahlführungseinheiten (10a, 10b, 10c, 10d) instrumentell unterschiedlich ausgelegt sind, derart, dass sie für unterschiedliche Bereiche der kinetischen Energie der Protonen und/oder für unterschiedliche Strahleigenschaften ausgelegt sind, indem die wenigstens eine Strahlablenkeinheit (14a, 14b) und/oder wenigstens eine Strahlformungseinheit (16a, 16b) einer jeweiligen der wenigstens zwei Strahlführungseinheiten (10a, 10b, 10c, 10d) an den jeweiligen Bereich der Energie der Protonen und/oder die jeweilige Strahleigenschaft des Protonenstrahls angepasst sind.

2. Protonenstrahl-Therapiesystem nach Anspruch 1,
**dadurch gekennzeichnet, dass** die wenigstens zwei Strahlführungseinheiten (10a, 10b, 10c, 10d) unterschiedliche Akzeptanzen aufweisen.

3. Protonenstrahl-Therapiesystem nach Anspruch 2,
**dadurch gekennzeichnet, dass** eine der wenigstens zwei Strahlführungseinheiten (10a, 10b, 10c, 10d) eine höhere Akzeptanz aufweist als eine andere der wenigstens zwei Strahlführungseinheiten (10a, 10b, 10c, 10d) und dass die Strahlführungseinheit mit der höheren Akzeptanz im Vergleich zu der anderen Strahlführungseinheit für Strahlen mit niedrigeren Teilchenenergien ausgelegt ist.

4. Protonenstrahl-Therapiesystem nach Anspruch 1, 2 oder 3,
**dadurch gekennzeichnet, dass** wenigstens eine der wenigstens zwei Strahlführungseinheiten (10a, 10b, 10c, 10d) auf eine optimale Strahltransmission ausgelegt ist.

5. Protonenstrahl-Therapiesystem nach einem der Ansprüche 1 bis 4,
**dadurch gekennzeichnet, dass** wenigstens eine der wenigstens zwei Strahlführungseinheiten (10a, 10b, 10c, 10d) auf eine optimale Strahlfokussierung ausgelegt ist.

6. Protonenstrahl-Therapiesystem nach Anspruch 1,
**dadurch gekennzeichnet, dass** die unterschiedlichen Strahleigenschaften unterschiedliche Phasenraumdichten oder unterschiedliche Breiten des Strahlprofils umfassen.

7. Protonenstrahl-Therapiesystem nach einem der Ansprüche 1 bis 6,
**dadurch gekennzeichnet, dass** die Strahlerzeugungseinheit (4) eine Beschleunigereinrichtung mit einstellbarer kinetischer Energie, insbesondere ein Synchrotron (6), aufweist.

8. Protonenstrahl-Therapiesystem nach einem der Ansprüche 1 bis 6,
**dadurch gekennzeichnet, dass** die Strahlerzeugungseinheit (4) eine Beschleunigereinrichtung mit konstanter kinetischer Energie, insbesondere ein Zyklotron (6'), und eine Energiekorrektureinheit, insbesondere einen Degrader (8'), aufweist.

9. Protonenstrahl-Therapiesystem nach Anspruch 8,
**dadurch gekennzeichnet, dass** der Degrader ein Material mit einer Ordnungszahl Z kleiner als 10 enthält.

10. Protonenstrahl-Therapiesystem nach einem der Ansprüche 1 bis 9,
**dadurch gekennzeichnet, dass** eine erste der wenigstens zwei Strahlführungseinheiten auf ein Energieintervall von 100 bis 250 MeV und eine zweite der wenigstens zwei Strahlführungseinheiten auf ein Energieintervall von 70 bis 200 MeV ausgelegt ist.

11. Protonenstrahl-Therapiesystem nach einem der Ansprüche 1 bis 10,
**dadurch gekennzeichnet, dass** es mehr als zwei Strahlführungseinheiten umfasst.

12. Protonenstrahl-Therapiesystem nach einem der Ansprüche 1 bis 10,
**dadurch gekennzeichnet, dass** es mehr als zwei Strahlführungseinheiten umfasst, darunter die wenigstens zwei instrumentell unterschiedlich ausgelegten Strahlführungseinheiten.

13. Protonenstrahl-Therapiesystem nach Anspruch 12,
**dadurch gekennzeichnet, dass** vier Strahlführungseinheiten vorhanden sind, von denen eine erste auf ein Energieintervall von 100 bis 250 MeV, eine zweite und dritte auf ein Energieintervall von 70 bis 200 MeV und eine vierte auf ein Energieintervall von 70 bis 150 MeV ausgelegt sind.

14. Verfahren zur rechnergestützten Steuerung eines Protonenstrahl-Therapiesystems nach einem der Ansprüche 1-13, umfassend:
- Zuordnen des Protonenstrahls zu einer der wenigstens zwei Strahlführungseinheiten (10a, 10b, 10c, 10d) anhand eines Zuordnungsalgorithmus gemäß der Energie der Protonen und/oder der Strahleigenschaften.

15. Verfahren nach Anspruch 14, wobei die Energie der Teilchen bzw. die Strahleigenschaften aus Einstellungen der Strahlerzeugungseinheit (4) abgeleitet und/oder mittels Monitoren am Protonenstrahl gemessen werden.

## Claims

1. Proton beam treatment system (2)
- with a beam generating unit (4) for generating and adjusting the kinetic energy of a proton beam,
- with at least two beam guide units (10a, 10b, 10c, 10d), each respectively having a rotatable isocentric gantry, for feeding the proton beam to a treatment location (26a, 26b) associated with the respective beam guide unit (10a, 10b, 10c, 10d)
- wherein the at least two beam guide units (10a, 10b, 10c, 10d) respectively comprise at least one beam deflection unit (14a, 14b) and/or at least one beam forming unit (16a, 16b),
**characterized in that**
the at least two beam guide units (10a, 10b, 10c, 10d) are of instrumentally different design, so that they are designed for different ranges of the kinetic energy of the protons and/or for different beam properties, such that the at least one beam deflection unit (14a, 14b) and/or the at least one beam forming unit (16a, 16b) of a respective one of the at least two beam guide units (10a, 10b, 10c, 10d) is adapted to the respective range of energy of the protons and/or to the respective beam property of the proton beam.

2. Proton beam treatment system as per claim 1, **characterized in that** the at least two beam guide units (10a, 10b, 10c, 10d) have different acceptances.

3. Proton beam treatment system as per claim 2, **characterized in that** a beam guide unit of the at least two beam guide units (10a, 10b, 10c, 10d) has a higher acceptance than another beam guide unit of the at least two beam guide units (10a, 10b, 10c, 10d) and that the beam guide unit with the higher acceptance is designed, in comparison to the other beam guide units, for beams with lower particle energies.

4. Proton beam treatment system as per claim 1, 2 or 3, **characterized in that** at least one of the at least two beam guide units (10a, 10b, 10c, 10d) is designed for optimum beam transmission.

5. Proton beam treatment system as per one of the claims 1 to 4, **characterized in that** at least one of the at least two beam guide units (10a, 10b, 10c, 10d) is designed for optimum beam focusing.

6. Proton beam treatment system as per claim 1, **characterized in that** the different beam properties comprise different phase space densities or different widths of the beam profile.

7. Proton beam treatment system as per one of the claims 1 to 6, **characterized in that** the beam generating unit (4) has an accelerator with adjustable kinetic energy, particularly a synchrotron (6).

8. Proton beam treatment system as per one of the claims 1 to 6, **characterized in that** the beam generating unit (4) has an accelerator with constant kinetic energy, particularly a cyclotron (6') and an energy correction unit, particularly a degrader (8').

9. Proton beam treatment system as per claim 8, **characterized in that** the degrader comprises a material with an atomic number Z smaller than 10.

10. Proton beam treatment system as per one of the claims 1 to 9, **characterized in that** a first one of the at least two beam guide units is designed for an energy interval from 100 to 250 MeV, and that a second one of the at least two beam guide units is designed for an energy interval from 70 to 200 MeV.

11. Proton beam treatment system as per one of the claims 1 to 10, **characterized in that** it comprises more that two beam guide units.

12. Proton beam treatment system as per one of the claims 1 to 10, **characterized in that** it comprises more that two beam guide units, including the two beam guide units that are of instrumentally different design.

13. Proton beam treatment system as per claim 12, **characterized in that** there are four beam guide units, of which a first one is designed for an energy interval from 100 to 250 MeV, a second one and a third one are designed for an energy interval from 70 to 200 MeV and a fourth one is designed for an energy interval from 70 to 150 MeV.

14. Method for computer-assisted control of a proton beam treatment system as per any of the claims 1-13, comprising:
- assigning the proton beam to a beam guide unit of the at least two beam guide units (10a, 10b, 10c, 10d) by means of an assignment algorithm according to the energy of the protons and/or the beam properties.

15. Method as per claim 14, wherein the energy of the particles and/or the beam properties are derived from settings of the beam generating unit (4) and/or are measured by means of monitors on the proton beam.

## Revendications

1. Système de thérapie par faisceau de protons (2)
- avec une unité de production de faisceau (4) pour la production et le réglage de l'énergie cinétique d'un faisceau de protons,
- avec au moins deux unités de guidage de faisceau (10a, 10b, 10c, 10d), présentant respectivement une Gantry iso-centrique rotative, pour l'amenée du faisceau de protons à un emplacement de traitement (26a, 26b) associé à l'unité de guidage de faisceau respective (10a, 10b, 10c, 10d),
- moyennant quoi les au moins deux unités de guidage de faisceau (10a, 10b, 10c, 10d) comprennent respectivement au moins une unité de déviation de faisceau (14a, 14b) et/ou au moins une unité de façonnage de faisceau (16a, 16b),
**caractérisé en ce que**
les au moins deux unités de guidage de faisceau (10a, 10b, 10c, 10d) sont étudiées différemment du point de vue instrumental de manière à être étudiées pour différentes plages d'énergie cinétique des protons et/ou pour différentes propriétés de faisceau **en ce que** l'au moins une unité de déviation de faisceau (14a, 14b) et/ou l'au moins une unité de façonnage de faisceau (16a, 16b) de respectivement l'une des au moins deux unités de guidage de faisceau (10a, 10b, 10c, 10d) sont adaptées à la plage respective de l'énergie des protons et/ou à la propriété de faisceau respective du faisceau de protons.

2. Système de thérapie par faisceau de protons selon la revendication 1,
**caractérisé en ce que**
les au moins deux unités de guidage de faisceau (10a, 10b, 10c, 10d) présentent des acceptances différentes.

3. Système de thérapie par faisceau de protons selon la revendication 2,
**caractérisé en ce que**
l'une des au moins deux unités de guidage de faisceau (10a, 10b, 10c, 10d) présente une acceptance plus haute qu'une autre des au moins deux unités de guidage de faisceau (10a, 10b, 10c, 10d) et **en ce que** l'unité de guidage de faisceau avec une acceptance plus haute en comparaison avec l'autre unité de guidage de faisceau est étudiée pour des faisceaux avec des énergies plus faibles des particules.

4. Système de thérapie par faisceau de protons selon les revendications 1, 2 ou 3,
**caractérisé en ce qu'**
au moins l'une des au moins deux unités de guidage de faisceau (10a, 10b, 10c, 10d) est étudiée pour une transmission optimale de faisceau.

5. Système de thérapie par faisceau de protons selon l'une des revendications 1 à 4,
**caractérisé en ce qu'**
au moins l'une des au moins deux unités de guidage de faisceau (10a, 10b, 10c, 10d) est étudiée pour une focalisation optimale du faisceau.

6. Système de thérapie par faisceau de protons selon la revendication 1,
**caractérisé en ce que**
les propriétés différentes du faisceau comprennent différentes densités d'espace de phase ou différentes largeurs du profil du faisceau.

7. Système de thérapie par faisceau de protons selon l'une des revendications 1 à 6,
**caractérisé en ce que**
l'unité de production de faisceau (4) présente un dispositif accélérateur avec une énergie cinétique réglable, en particulier un synchrotron (6).

8. Système de thérapie par faisceau de protons selon l'une des revendications 1 à 6,
**caractérisé en ce que**
l'unité de production de faisceau (4) présente un dispositif accélérateur avec une énergie cinétique constante, en particulier un cyclotron (6'), et une unité de correction d'énergie, en particulier un dégradeur (8').

9. Système de thérapie par faisceau de protons selon la revendication 8,
**caractérisé en ce que**
le dégradeur contient un matériau avec un numéro atomique Z inférieur à 10.

10. Système de thérapie par faisceau de protons selon l'une des revendications 1 à 9,
**caractérisé en ce qu'**
une première des au moins deux unités de guidage de faisceau est étudiée pour un intervalle d'énergie de 100 à 250 MeV et **en ce qu'**une deuxième des au moins deux unités de guidage de faisceau est étudiée pour un intervalle d'énergie de 70 à 200 MeV.

11. Système de thérapie par faisceau de protons selon l'une des revendications 1 à 10,
**caractérisé en ce qu'**
il comprend plus de deux unités de guidage de faisceau.

12. Système de thérapie par faisceau de protons selon l'une des revendications 1 à 10,
**caractérisé en ce qu'**
il comprend plus de deux unités de guidage de faisceau, entre autres les au moins deux unités de guidage de faisceau étudiées différemment du point de vue instrumental.

13. Système de thérapie par faisceau de protons selon la revendication 12,
**caractérisé en ce qu'**
il y a quatre unités de guidage de faisceau, une première d'entre elles étant étudiée pour un intervalle d'énergie de 100 à 250 MeV, une deuxième et une troisième pour un intervalle d'énergie de 70 à 200 MeV et une quatrième pour un intervalle d'énergie de 70 à 150 MeV.

14. Procédé pour la commande assistée par ordinateur d'un système de thérapie par faisceau de protons selon l'une des revendications 1-13, comprenant :
- association du faisceau de protons à l'une des au moins deux unités de guidage de faisceau (10a, 10b, 10c, 10d) à l'aide d'un algorithme d'association conformément à l'énergie des protons et/ou aux propriétés du faisceau.

15. Procédé selon la revendication 14, dans lequel l'énergie des particules ou les propriétés de faisceau sont déduites à partir de réglages de l'unité de production de faisceau (4) et/ou sont mesurées à l'aide de moniteurs au niveau du faisceau de protons.
